# EUROPÄISCHE PATENTSCHRIFT

(11) **EP 1 772 110 B1**
(45) Veröffentlichungstag und Bekanntmachung des Hinweises auf die Patenterteilung: **17.03.2010**
(21) Anmeldenummer: 05021914.6
(22) Anmeldetag: 07.10.2005
(51) Int. Cl.: A61B 19/00

(54) **Medizintechnische Markereinrichtung**
Medical marker
Marqeur médical

(43) Veröffentlichungstag der Anmeldung: 11.04.2007
(73) Patentinhaber: BrainLAB AG, 85622 Feldkirchen (DE)
(72) Erfinder: Birkenbach, Rainer, 85445 Aufkirchen (DE)
(74) Vertreter: Schwabe - Sandmair - Marx

(56) Entgegenhaltungen:
- WO-A-00/39576
- WO-A-01/47438
- US-A- 3 800 132
- US-A- 4 015 111
- US-A- 5 350 391
- US-A- 5 860 845
- US-A1- 2002 065 019
- US-A1- 2002 151 784
- US-A1- 2003 180 800
- US-A1- 2004 082 263
- US-B1- 6 485 158
- US-B1- 6 782 289
- PATENT ABSTRACTS OF JAPAN vol. 2003, no. 12, 5 December 2003 (2003-12-05) & JP 2005 011538 A (OMNIGLOW JAPAN CO LTD), 13 January 2005 (2005-01-13)

## Beschreibung

Die Erfindung betrifft eine medizintechnische Markereinrichtung mit einem Markerkörper, der von einem medizintechnischen Ortungs- bzw. Lokalisations- oder Trackingsystem positionell detektiert werden kann. Solche Markereinrichtungen werden entweder als passive Marker oder als aktive Marker beispielsweise im Rahmen der chirurgischen Navigation von Instrumenten und Behandlungseinrichtungen verwendet. Aktive Marker - beispielsweise LED-Marker - haben den Nachteil, dass sie von außen mit Energie versorgt werden müssen. Instrumente, die mit solchen Markern ausgestattet sind, haben entweder ein Kabel oder werden durch Batterien oder Akkus mit Energie versorgt. Ein weiterer Nachteil von LEDs ist der schmale Abstrahlwinkel.

Kabel stören bei der Handhabung und Batterien bzw. Akkus sind schwer zu sterilisieren und auch schwer im Gewicht.. Passive Marker, wie sie beispielsweise aus der DE 196 39 615 A1 bekannt sind, lösen die Probleme der schlechten Handhabbarkeit sowie der Energieversorgung (Sterilisierung). Ihre Herstellung mit reflektierenden Überzügen ist aber relativ kompliziert und erfordert viel Handarbeit.

Aus der US 2003/0181800 A1 ist ein Verfahren zum Sichern von Körpergewebe bekannt, das mit Hilfe eines Robotmechanismus durchgeführt wird. Dabei werden lumineszente Marker zur besseren Sichtbarmachung im Körperinneren des Patienten verwendet, wobei die Marker an sichtbar zu machenden Gerätschaften im Inneren des Patientenkörpers angeordnet werden.

Die WO 00/39576 offenbart ein System, das die dreidimensionale Position und Ausrichtung einer Sonde mit Hilfe mehrerer Sensoren trackt. Aus der US 6,782,289 B1 ist die Charakterisierung von Läsionen in Blutgefäßen und anderen Körperlumen bekannt, wobei intraluminale Detektoren verwendet werden, die radioaktive oder andere Kennzeichnungen erfassen. Die US 5,350,391 schlägt vor, an einem laparoskopischen chirurgischen Instrument gefärbte Streifen anzubringen, um die Schnitttiefe besser bestimmen zu können. Aus der US 6,485,158 B1 ist ein aufblasbares chemolumineszentes System zum Beleuchten von Ballons mit Hilfe einer chemischen Reaktion bekannt.

Es ist die Aufgabe der vorliegenden Erfindung, eine Markereinrichtung bereitzustellen, welche die Vorteile der passiven Marker bietet, eine ausreichende Sichtbarkeit bereitstellt und insbesondere auch einfach herstellbar ist.

Diese Aufgabe wird erfindungsgemäß durch eine Markereinrichtung gemäß dem Anspruch 1 gelöst. Die Unteransprüche definieren bevorzugte Ausführungsformen der Erfindung.

Gemäß der vorliegenden Erfindung umfasst der Markerkörper ein ohne äußere Energieversorgung zum Leuchten bringbares Lumineszenzmittel. Mit anderen Worten greift die Erfindung auf selbstleuchtende bzw. kaltleuchtende Mittel zurück, die der Markereinrichtung die Möglichkeit geben, vom Material her selbst zu leuchten, ohne dass eine Energieversorgung bereitgestellt werden muss. Vorteilhafterweise erspart man sich so die aufwändige Herstellung von reflektiven Überzügen für die Marker, und man schafft leicht sterilisierbare Systeme mit guter Leuchtkraft, das heißt guter Erkennungsfähigkeit für die jeweilige Markereinrichtung. Ein weiterer Vorteil ist, dass man die IR-Beleuchtung (in der Kamera eingebaut) des Umfeldes einsparen kann; diese braucht viel Strom und stört gelegentlich andere OP-Geräte wie Videorekorder-Fernsteuerungen oder auch OP-Tisch Fernsteuerungen oder Finger-Pulsmesser.

Wie oben schon angesprochen wurde, ist mit dem Begriff "Lumineszenz" im vorliegenden Kontext im wesentlichen gemeint, dass der Markerkörper nur aus dem verwendeten Materialien bzw. dem verwendeten Material heraus leuchten kann, ohne eine äußere Energieversorgung zu benötigen (Kabel oder angeschlossene Batterien/Akkus).

Das angesprochene Lumineszenzmittel kann eines sein, das nach mindestens einem der folgenden Prozesse luminesziert: Chemolumineszenz (eine chemische Reaktion liefert die Energie, um Elektronen in höhere Energiezustände zu heben), Fotolumineszenz (Elektronen werden durch optische Anregung (Bestrahlung) in den höheren Energiezustand gebracht), Elektrolumineszenz (die Emission von Licht wird durch das Anlegen eines elektrischen Feldes hervorgerufen) und/oder Radiolumineszenz (Einschuss von Teilchen in ein geeignetes Material zur Erzeugung von Lumineszenz, zum Beispiel Elektronen, Alpha-Teilchen).

Im Folgenden wird sich die vorliegende Beschreibung erfindungsgemäss mit der Verwendung der Chemolumineszenz beschäftigen. Es soll aber ausdrücklich festgestellt werden, dass es durchaus denkbar ist, andere Lumineszenzarten zu verwenden, und insbesondere ist hier als Beispiel auch die Fotolumineszenz hervorzuheben. Es ist durchaus denkbar, Markereinrichtungen vorab mit geeigneten Lichtmengen zu bestrahlen, dann an Instrumenten oder Behandlungseinrichtungen zu positionieren und über die Zeit zu verwenden, in der sie die Lichtenergie wieder abstrahlen (wie beispielsweise die Leuchtziffern einer Uhr).

Die oben angesprochene Chemolumineszenz ist eine mit einer chemischen Reaktion verbundene Lumineszenz, das heißt die Emission von Licht im sichtbaren Bereich aber auch von ultraviolettem oder infrarotem Licht. Die Temperatur liegt dabei deutlich unterhalb der Glühtemperatur der beteiligten Substanzen und man spricht deshalb auch von "kaltem Licht". Gerade letztere Eigenschaft macht die Chemolumineszenz sehr geeignet für medizintechnische Anwendungen; es wird keine störende, überschüssige oder abzuführende Wärme erzeugt.

Bei der Chemolumineszenz wird elektrische Energie in elektronische oder seltener in Schwingungs-Energie verwandelt. Dabei wird vorausgesetzt, dass diese Energiefreisetzung auf einmal, das heißt, nicht in zahlreichen Stufen erfolgt. Chemolumineszenz tritt bei zahlreichen chemischen Prozessen auf, in denen energiereiche, instabile Zwischenstufen entstehen und sofort wieder zerfallen. Obwohl die Aufklärung des Reaktionsmechanismus vieler Chemolumineszenz-Reaktionen noch nicht endgültig abgeschlossen ist, wurde das Konzept der Bildung energiereicher Vierringsysteme (Dioxetane) in vielen Fällen experimentell bestätigt. Durch Reaktion von Sauerstoff (O₂), Peroxiden (R₂O₂), Hyperoxiden (O₂⁻) oder Hydroperoxiden (RO₂H) mit Luminophoren entstehen oft Dioxetane, wie 1,2-Dioxitane oder 1,2-Dioxete, die ihrerseits in zwei Carbonylverbindungen zerfallen. Dabei entsteht eine von ihnen im elektronisch angeregten Singulett- oder TriplettZustand. Das Bruchstück im elektronisch angeregten Zustand kann durch Abgabe der Energie in Form eines Lichtquanten in den Grundzustand zurückkehren. Während die Molekühle im angeregten Singulett-Zustand schell unter Aussendung des charekteristischen Fluoreszenzlichts in den Grundzustand zurückkehren, sind die angeregten Triplet-Moleküle längerlebig. Das somit in wesentlich geringerer Ausbeute entstehende Phosphoreszenzlicht leistet kaum einen Beitrag zur "verwertbaren" Chemolumineszenz.

Ein Beispiel für eine Chemolumineszenz ist die sogenannte "Luminol-Reaktion", ein Beispiel für einen Oxidationsprozess, bei dem die Reaktionsenergie nicht als Wärme, sondern ausschließlich als Lichtenergie abgestrahlt wird. Die Reaktion beruht auf der oxidativen Freisetzung von Stickstoff aus Phtalsäurehydrazit (Luminol) durch Einwirkung von alkalischem Wasserstoffperoxid. Diese Reaktion wird durch rotes Blutlaugensalz (Hexacyanoferrat (III)) katalisiert. Die Lumineszenz kann nicht nur durch Wasserstoff-Peroxid sondern auch durch Ozon ausgelöst werden. Luminol zeigt in alkalischer Wasserstoff-Peroxid-Lösung eine schwache, jedoch lang anhaltende Chemolumineszenz, deren Intensität durch bestimmte Katalisatoren (Kalziumhexacyanoferrat (III), Hämin) verstärkt wird, und zwar unter gleichzeitiger Verminderung der Abglimmzeit.

Bekannt ist die Chemolumineszenz auch durch Leuchtstäbe (Knicklichter). Hierbei enthält eine Glasampulle Sauerstoffperoxid, das als Oxidationsmittel für die Reaktion verwendet wird, welche den Stab zum Leuchten bringt. Wenn die Sollbruchstelle in der Ampulle gebrochen wird, wird das Wasserstoffperoxid in eine Lösung hinein freigegeben, die Oxalphtalatester enthält. Wenn dieses Oxalphtalatester oxidiert um Phenol und Kohlendioxid zu bilden, wird die Zwischenstufe 1,2-Dioxetane-3,4-Dione gebildet. Diese Zwischenstufe reagiert mit Farbstoffmolekülen im Leuchtstab, typischerweise mit einem Diphenyl-Antracen, und das Farbstoffmolekül wird elektronisch angeregt. In diesem angeregten Zustand emittiert es ein Photon und erzeugt so die Lumineszenz.

Die oben beschriebene oder ähnliche Chemolumineszenzen werden also erfindungsgemäß so eingesetzt, dass das Lumineszenzmittel aus mehreren Substanzen besteht, die ausgelegt sind, durch Mischen zum Leuchten gebracht zu werden. Die Vermischung selbst kann dabei separat ausgelöst werden, und zwar durch ein geeignetes Auslösemittel. Andererseits besteht die Möglichkeit, Markereinrichtungen zu verwenden, die eine Befestigung aufweisen, mit der sie an Instrumenten oder Behandlungseinrichtungen angebracht werden können, und die ausgelegt ist, das Mischen der Substanzen durch die Betätigung oder die Fixierung der Befestigung auszulösen. In diesem Fall, aber auch in Fällen, wo eine separate Auslösung erfolgt, kann der Markerkörper einen Behälter für eine erste Substanz bilden, in welcher ein weiterer Behälter angeordnet ist, der eine zweite Substanz aufweist. Beispielsweise durch das Brechen des inneren Behälters wird dann das Mischen der Substanzen ausgelöst. Wenn die oben beschriebene Befestigung verwendet wird, kann diese ausgelegt sein, bei ihrer Fixierung einen Behälter mit einer ersten Substanz zu öffnen, der sich innerhalb eines Raumes befindet, der eine zweite Substanz aufweist.

Für Navigations- und Trackingssysteme ist es oft von Vorteil, wenn sie im Infrarotlichtbereich arbeiten, damit Störungen durch sichtbares Licht ausgeschlossen werden können. Deshalb ist gemäß einer Ausführungsform der vorliegenden Erfindung das Lumineszenzmittel ein im Infrarotbereich strahlendes Lumineszenzmittel. Ferner kann das Lumineszenzmittel ein Mittel sein, das allein oder wenigstens teilweise und vorzugsweise nicht blendend im Bereich des sichtbaren Lichts strahlt. Die Markereinrichtung kann einen Indikator aufweisen, der anzeigt, wenn die Leuchtkraft des Lumineszenzmittels abnimmt oder versiegt. Bei einer speziellen Ausführungsform kann dieser Indikator der im Bereich des sichtbaren Lichts strahlende Leuchtanteil eines ansonsten im Infrarotbereich strahlenden Lumineszenzmittels sein. Mit anderen Worten würde man einen Markerkörper bereitstellen, dessen Lumineszenzmittel im Infrarotbereich leuchtet, aber auch in geringem Maße über einen begrenzten Wellenlängenbereich mit sichtbarem Licht. Wellenlängen für IR-Licht sind z.B. 700-900 nm, speziell 850-890 nm; das sichtbare Licht kann unter 700 nm Wellenlänge liegen. Chemolumineszente Emitter, die im Infrarotbereich strahlen können, währen beispielsweise CH₃SE (750 bis 825 nm Wellenlänge), IF (450 bis 800 nm) und SF₂ (550 bis 875 nm). Anhand des geringen Anteils sichtbaren Lichts könnte aber festgestellt werden, wenn der Marker in der Leuchtkraft abnimmt oder versiegt, und er könnte durch einen neuen Marker ersetzt werden.

Die Markereinrichtung muss nicht aus einem einzelnen Marker bestehen, sie kann auch mehrere Markerkörper oder mehrere Gruppen von Markerkörpern umfassen, deren Lumineszenzmittel insbesondere jeweils in einer anderen Farbe leuchtet. Wenn dies der Fall ist, können die Markerkörper oder Markerkörpergruppen in einem System mit einem Instrument oder einer Behandlungseinrichtung durch die Lumineszenzmittel farbkodiert und charakteristisch für das jeweilige Instrument oder die jeweilige Behandlungseinrichtung an dem Instrument oder an der Behandlungseinrichtung vorgesehen bzw. angebracht sein. Dabei kann das Instrument bzw. die Behandlungseinrichtung Markerkörper oder Markerkörpergruppen in derselben Farbe oder in unterschiedlichen Farben aufweisen.

Gemäß einer weiteren Ausführungsform der vorliegenden Erfindung mit einem System aus Markereinrichtung und einem Instrument oder einer Behandlungseinrichtung bildet der Markerkörper einen Einschub, der in eine Aufnahme eines Instruments oder einer Behandlungseinrichtung einschiebbar ist, wobei in oder an der Aufnahme Durchscheinöffnungen für das vom Markerkörper abgestrahlte Licht vorgesehen sind. Vorteilhafterweise könnten dabei für stabförmige Instrumente beispielsweise schon vorhandene stabförmige Lumineszenz-Leuchtmittel verwendet werden. Als Markereinrichtung selbst würden dann die Durchscheinöffnungen dienen, die natürlich sehr billig und einfach herstellbar sind.

Die Erfindung wird im Weiteren anhand von Ausführungsformen näher erläutert. Sie kann alle hierin beschriebenen Merkmale einzeln und in jedweder Kombination aufweisen. In den Zeichnungen zeigen:
- Figur 1: einen erfindungsgemäßen Lumineszenzmarker vor dem Befestigen an einem chirurgischen Instrument;
- Figur 2: den Marker aus Figur 1, am Instrument befestigt; und
- Figur 3: eine Ausführungsform der Erfindung mit einschiebbarem Lumineszenzstab-Marker.

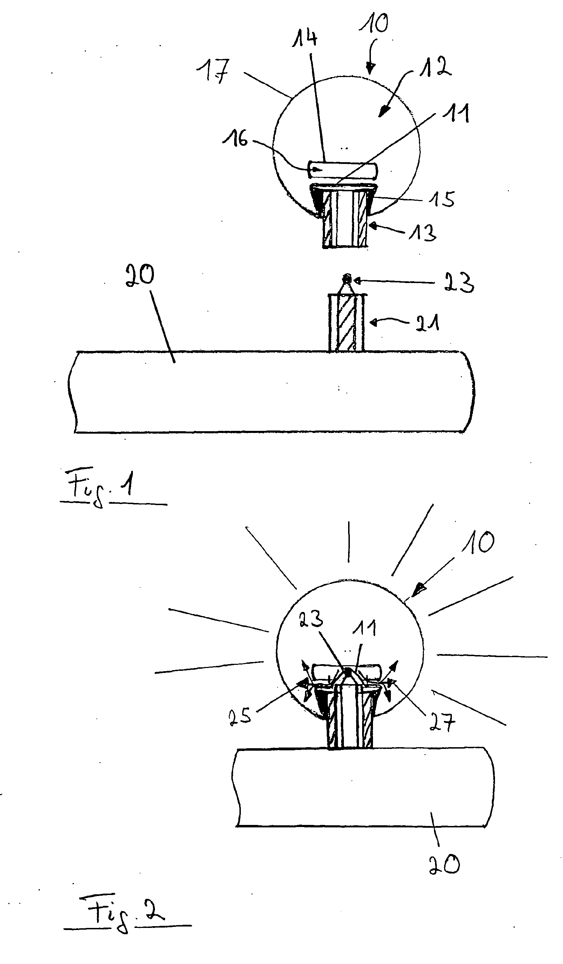

Die Figuren 1 und 2 zeigen zusammen ein Marker-Befestigungssystem zum Anbringen eines Markers 10 an einem chirurgischen Instrument 20. Der Marker 10 weist eine lichtdurchlässige bzw. diffus lichtdurchlässige Umhüllung auf, die kugelförmig ausgebildet ist. Der kugelförmige Teil 17 der Umhüllung schließt an ein Verbindungsstück 15 an, das wiederum an die Membran 11 anschließt. Zusammen bilden die Teile 11, 15 und 17 einen abgeschlossenen Raum. Mit dem Verbindungsstück 15 ist die Umhüllung mit dem ersten Befestigungsteil der Buchse 13 verbunden, das ein Innengewinde aufweist, welches sich in einer zentralen Innen-Durchgangsbohrung erstreckt.

Ferner ist über der Membran 11 noch eine Kapsel 14" aus einem brechbaren Material fixiert.

Während eine erste Flüssigkeit 12 sich in der kugelförmigen Umhüllung 17 befindet, trägt die Kapsel 14 eine zweite Flüssigkeit 16. Die beiden Flüssigkeiten sind Substanzen, die dann, wenn sie miteinander vermischt werden, eine Chemolumineszenz erzeugen und vorzugsweise hauptsächlich im infraroten Längenbereich strahlen (um 860 nm-890 nm). Außerdem kann diese Strahlung noch einen geringen Anteil an sichtbarem Licht umfassen. Es können die oben beschriebenen Substanzen oder jedwede Substanzen, die Fachleuchte als geeignet ansehen würden, verwendet werden.

Auf der Seite des chirurgischen Instruments 20, von dem nur ein Teil des Griffes zu sehen ist, befindet sich der zweite Befestigungsteil, nämlich ein hervorstehender Stift 21 mit einem Außengewinde. Auf dem Oberteil des Stiftes 21 ist die Brechvorrichtung schematisch als abgerundete Spitze 23 angedeutet.

Die Figur 2 zeigt nun, was passiert, wenn der Marker 10 mit seinem ersten Befestigungsteil 13 auf das zweite Befestigungsteil, nämlich den Stift 21 aufgeschraubt wird. Nach dem Aufschrauben ragt die Spitze 23 über den oberen Rand der Buchse 13 hinaus und verlängert dabei die Membran 11, ohne sie zu beschädigen. Die Spitze 23 zerstört durch die Membran das brechbare Material der Kapsel 14 und sorgt so dafür, dass die zweite Flüssigkeit 16 aus der Kapsel 14 austreten und sich mit der ersten Flüssigkeit 12 im kugelförmigen Marker vermischen kann. Angedeutet ist der Austritt der Flüssigkeit durch die Pfeile 25 und 27 in Figur 2. Beim Vermischen der Flüssigkeiten entsteht die Lumineszenz, und der Marker 10 kann so als Ortungshilfe für das Instrument 20 über die gesamte Lumineszenzdauer eingesetzt werden. Wenn die Leuchtkraft abnimmt oder versiegt, sieht der Verwender dies an der Abnahme bzw. am Versiegen des sichtbaren Lichtanteils, und der Marker kann ausgetauscht werden, indem er abgeschraubt und durch einen anderen ersetzt wird. Weil die Membran 11 beim Zerbrechen der Ampulle 14 nicht zerstört wird, tritt keine Flüssigkeit aus dem Markerinnenraum aus; das System bleibt "sauber" und die Marker können als vorsterilisierte Wegwerfartikel bereitgestellt werden.

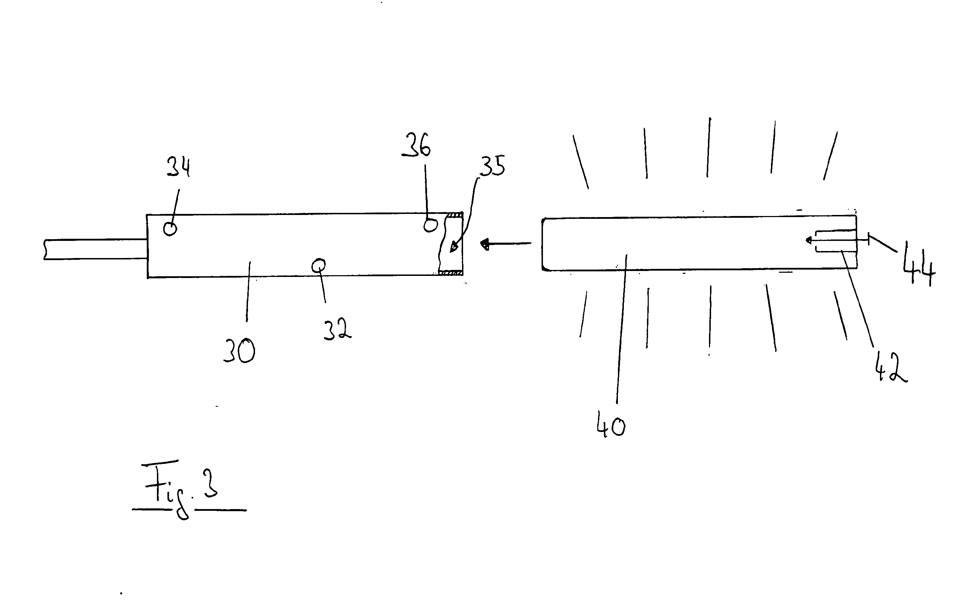

Eine weitere Ausführungsform der Erfindung ist in Figur 3 dargestellt. Die Figur 3 zeigt links einen Instrumentengriff 30, der an verschiedenen Stellen Durchgangslöcher 32, 34, 36 umfasst. Der Griff 30 ist innen hohl ausgebildet, wie durch das weggeschnittene rechte Stirnende sichtbar wird; er weist die Aufnahme 35 auf.

In diese Aufnahme 35 kann ein Lumineszenzstab 40 eingeführt werden, der rechts neben dem Griff 30 dargestellt ist. Der Lumineszenzstab 40 weist wiederum einen abgeschlossenen Innenraum mit einer ersten Substanz und eine zerbrechbare Ampulle 42 mit einer zweiten Substanz darin auf. Mit dem Bezugszeichen 44 ist schematisch eine Betätigungsvorrichtung aufgezeigt, mit der das Stirnende der Ampulle 42 zerbrochen und damit das Mischen der Substanzen und die Lumineszenz ausgelöst werden kann.

Mit der Betätigungsvorrichtung 44 kann vor dem Einschieben des Lumineszenzstabes 40 das Vermischen der beiden Flüssigkeiten in diesem ausgelöst werden, und danach lässt sich der Stab 40 einfach von hinten in die Aufnahme 35 des Griffs 30 einschieben. Daraufhin scheint der Stab 40 durch seine Lumineszenz durch die Durchscheinöffnungen (Löcher) 32, 34 und 36 hindurch und schafft somit eine Art Markeranordnung bzw. Markergruppe aus drei Öffnungen, die von einem Trackingsystem erkannt werden kann. Auch hier ist Platz für Individualisierungen; jedes Instrument kann in charakteristischer Weise angeordnete Durchscheinöffnungen aufweisen, damit das Tracking- bzw. Navigationssystem es erkennen und zuordnen kann. Auf diese Art lässt sich auf sehr einfache Weise, insbesondere möglicherweise mit einem schon handelsüblich erhältlichen Lumineszenzstab, ein identifizierbares chirurgisches Instrument mit einer Markeranordnung herstellen.

## Patentansprüche

1. Medizinische Markereinrichtung mit einem Markerkörper (10), der von einem medizintechnischen Ortungs- bzw. Lokalisations- oder Trackingsystem positionell detektiert werden kann, wobei der Markerkörper (10) ein ohne äußere Energieversorgung zum Leuchten bringbares Lumineszenzmittel (12, 16) umfasst, **dadurch gekennzeichnet, dass** das Lumineszenzmittel aus mehreren Substanzen (12, 16) besteht, die ausgelegt sind, durch Mischen zum Leuchten gebracht zu werden.

2. Medizinische Markereinrichtung nach Anspruch 1, **dadurch gekennzeichnet, dass** das Lumineszenzmittel eines ist, das nach mindestens einem der folgenden Prozessen luminesziert: Chemolumineszenz, Photolumineszenz, Elektrolumineszenz, Thermolumineszenz und/oder Radiolumineszenz.

3. Medizinische Markereinrichtung nach Anspruch 2, **dadurch gekennzeichnet, dass** sie eine Befestigung (13, 21, 23) aufweist, die ausgelegt ist, das Mischen der Substanzen (12, 16) durch die Betätigung oder die Fixierung der Befestigung (13, 21, 23) auszulösen.

4. Medizinische Markereinrichtung nach Anspruch 3, **dadurch gekennzeichnet, dass** die Befestigung ausgelegt ist, bei ihrer Fixierung einen Behälter (14) mit einer ersten Substanz (16) zu öffnen, der sich innerhalb eines Raumes befindet, der eine zweite Substanz (12) aufweist.

5. Medizinische Markereinrichtung nach einem der Ansprüche 1 bis 4, **dadurch gekennzeichnet, dass** das Lumineszenzmittel (12, 16) ein im Infrarotbereich strahlendes Lumineszenzmittel ist.

6. Medizinische Markereinrichtung nach einem der Ansprüche 1 bis 5, **dadurch gekennzeichnet, dass** das Lumineszenzmittel (12, 16) ein Mittel ist, das allein oder wenigstens teilweise und vorzugsweise nicht blendend im Bereich des sichtbaren Lichts strahlt.

7. Medizinische Markereinrichtung nach einem der Ansprüche 1 bis 6 **dadurch gekennzeichnet, dass** sie einen Indikator aufweist, der anzeigt, wenn die Leuchtkraft des Lumineszenzmittels (12, 16) abnimmt oder versiegt.

8. Medizinische Markereinrichtung nach Anspruch 6 oder 7, **dadurch gekennzeichnet, dass** der im Bereich des sichtbaren Lichts strahlende Leuchtanteil eines auch im Infrarotbereich strahlenden Lumineszenzmittels (12, 16) den Indikator bildet.

9. Medizinische Markereinrichtung nach einem der Ansprüche 1 bis 8, **dadurch gekennzeichnet, dass** sie mehrere Markerkörper oder mehrere Gruppen von Markerkörpern umfasst, deren Lumineszenzmittel insbesondere jeweils in einer anderen Farbe leuchtet.

10. System aus einer medizinischen Markereinrichtung nach Anspruch 9, und einem Instrument oder einer Behandungseinrichtung **dadurch gekennzeichnet, dass** die Markerkörper oder Markerkörpergruppen durch die Lumineszenzmittel farbcodiert und charakteristisch für das jeweilige Instrument (20) oder die jeweilige Behandlungseinrichtung an dem Instrument oder an der Behandlungseinrichtung angebracht sind.

11. System nach Anspruch 10, **dadurch gekennzeichnet, dass** ein Instrument Markerkörper oder Markerkörpergruppen in derselben Farbe aufweist.

12. System nach Anspruch 10, **dadurch gekennzeichnet, dass** ein Instrument Markerkörper oder Markerkörpergruppen in unterschiedlichen Farben aufweist.

13. System aus einer medizinischen Markereinrichtung nach einem der Ansprüche 1 bis 12 und einem Instrument oder einer Behandlungseinrichtung, **dadurch gekennzeichnet, dass** der Markerkörper (40) einen Einschub bildet, der in eine Aufnahme (35) des Instruments (30) oder der Behandlungseinrichtung einschiebbar ist, wobei in oder an der Aufnahme (35) Durchscheinöffnungen (32, 34, 36) für das vom Markerkörper (40) abgestrahlte Licht vorgesehen sind.

## Claims

1. A medical marker means comprising a marker body (10) which can be positionally detected by a medical localisation or tracking system, wherein the marker body (10) comprises a luminescence agent (12, 16) which can be illuminated without an external energy supply, **characterised in that** the luminescence agent consists of a number of substances (12, 16) which are designed such that they are illuminated by being mixed.

2. The medical marker means according to claim 1, **characterised in that** the luminescence agent is one which luminesces according to at least one of the following processes: chemoluminescence; photoluminescence; electroluminescence; thermoluminescence; and/or radioluminescence.

3. The medical marker means according to claim 2, **characterised in that** it comprises a fastening (13, 21, 23) which is designed such that the mixing of the substances (12, 16) is triggered by activating or fixing the fastening (13, 21, 23).

4. The medical marker means according to claim 3, **characterised in that** the fastening is designed such that fixing it opens a container (14) which comprises a first substance (16) and is situated within a space comprising a second substance (12).

5. The medical marker means according to any one of claims 1 to 4, **characterised in that** the luminescence agent (12, 16) is a luminescence agent which emits in the infrared range.

6. The medical marker means according to any one of claims 1 to 5, **characterised in that** the luminescence agent (12, 16) is an agent which solely or at least partially and preferably not glaringly emits in the range of visible light.

7. The medical marker means according to any one of claims 1 to 6, **characterised in that** it comprises an indicator which shows when the luminosity of the luminescence agent (12, 16) decreases or ebbs.

8. The medical marker means according to claim 6 or 7, **characterised in that** the illuminating portion, emitting in the range of visible light, of a luminescence agent (12, 16) which also emits in the infrared range forms the indicator.

9. The medical marker means according to any one of claims 1 to 8, **characterised in that** it comprises a number of marker bodies or a number of groups of marker bodies, the luminescence agent of which in particular illuminates in a different colour in each case.

10. A system consisting of a medical marker means according to claim 9 and an instrument or treatment means, **characterised in that** the marker bodies or groups of marker bodies are attached to the instrument or treatment means in a way which is colour-coded by the luminescence agents and characteristic of the respective instrument (20) or treatment means.

11. The system according to claim 10, **characterised in that** an instrument comprises marker bodies or groups of marker bodies in the same colour.

12. The system according to claim 10, **characterised in that** an instrument comprises marker bodies or groups of marker bodies in different colours.

13. A system consisting of a medical marker means according to any one of claims 1 to 12 and an instrument or treatment means, **characterised in that** the marker body (40) forms an insert which can be inserted into a receptacle (35) of the instrument (30) or treatment means, wherein translucent openings (32, 34, 36) for the light emitted by the marker body (40) are provided in or on the receptacle (35).

## Revendications

1. Marqueur médical comportant un corps marqueur (10) dont la position peut être détectée par un système médicotechnique de repérage, de localisation ou de suivi, dans lequel le corps marqueur (10) comporte un agent de luminescence (12, 16) pouvant s'illuminer sans apport d'énergie externe, **caractérisé en ce que** l'agent de luminescence est constitué de plusieurs substances (12, 16) qui sont adaptées pour s'illuminer lorsqu'elles sont mélangées.

2. Marqueur médical selon la revendication 1, **caractérisé en ce que** la luminescence de l'agent de luminescence est obtenue par au moins un des processus suivants : chimioluminescence, photoluminescence, électroluminescence, thermoluminescence et/ou radioluminescence.

3. Marqueur médical selon la revendication 2, **caractérisé en ce qu'**il comporte un dispositif de fixation (13, 21, 23) qui est adapté pour déclencher le mélange des substances (12, 16) lorsque le dispositif de fixation (13, 21, 23) est actionné ou fixé.

4. Marqueur médical selon la revendication 3, **caractérisé en ce que** le dispositif de fixation est adapté de manière à ouvrir, lors de sa fixation, un conteneur (14) incluant une première substance (16), ledit conteneur étant situé dans un espace comportant une seconde substance (12).

5. Marqueur médical selon l'une quelconque des revendications 1 à 4, **caractérisé en ce que** l'agent de luminescence (12, 16) est un agent de luminescence rayonnant dans le domaine de l'infrarouge.

6. Marqueur médical selon l'une quelconque des revendications 1 à 5, **caractérisé en ce que** l'agent de luminescence (12, 16) est un agent qui rayonne seul ou au moins partiellement et de préférence de manière non éblouissante dans le domaine de la lumière visible.

7. Marqueur médical selon l'une quelconque des revendications 1 à 6, **caractérisé en ce qu'**il comporte un indicateur qui signale lorsque la luminosité de l'agent de luminescence (12, 16) diminue ou disparaît.

8. Marqueur médical selon la revendication 6 ou 7, **caractérisé en ce que** la partie lumineuse rayonnant dans le domaine de la lumière visible d'un agent de luminescence (12, 16) rayonnant également dans le domaine de l'infrarouge forme l'indicateur.

9. Marqueur médical selon l'une quelconque des revendications 1 à 8, **caractérisé en ce qu'**il comporte plusieurs corps marqueurs ou plusieurs groupes de corps marqueurs, l'agent de luminescence s'illuminant en particulier dans une autre couleur dans chaque groupe ou corps.

10. Système constitué d'un marqueur médical selon la revendication 9 et d'un instrument ou des moyens de traitement, **caractérisé en ce que** les corps marqueurs ou les groupes de corps marqueurs sont codés par couleur au moyen de l'agent de luminescence, et sont fixés sur l'instrument ou sur les moyens de traitement de manière caractéristique à l'instrument respectif (20) ou aux moyens de traitement respectifs.

11. Système selon la revendication 10, **caractérisé en ce qu'**un instrument comporte des corps marqueurs ou des groupes de corps marqueurs de couleur identique.

12. Système selon la revendication 10, **caractérisé en ce qu'**un instrument comporte des corps marqueurs ou des groupes de corps marqueurs de différentes couleurs.

13. Système constitué d'un marqueur médical selon l'une quelconque des revendications 1 à 12 et d'un instrument ou de moyens de traitement, **caractérisé en ce que** le corps marqueur (40) forme un élément d'insertion qui peut être introduit dans un logement (35) de l'instrument (30) ou des moyens de traitement, dans lequel des ouvertures translucides (32, 34, 36) sont prévues dans ou sur le logement (35) pour la lumière émise par le corps marqueur (40).
